# EUROPÄISCHE PATENTSCHRIFT

(11) **EP 1 188 416 B1**
(45) Veröffentlichungstag und Bekanntmachung des Hinweises auf die Patenterteilung: **01.06.2005**
(21) Anmeldenummer: 01810816.7
(22) Anmeldetag: 22.08.2001
(51) Int. Cl.: A61B 17/70

(54) **Pedikelschraube für intervertebrale Stützelemente**
Pedicle screw for intervertebral support element
Vis pédiculaire pour élément de support intervertébral

(30) Priorität: 18.09.2000 EP 00810845
(43) Veröffentlichungstag der Anmeldung: 20.03.2002
(73) Patentinhaber: Zimmer GmbH, 8404 Winterthur (CH)
(72) Erfinder: Walder, Reto, 8422 Pfungen (CH); Braunschweiler, Reto, 8413 Neftenbach (CH)
(74) Vertreter: Manitz, Finsterwald & Partner Gbr

(56) Entgegenhaltungen:
- EP-A- 0 669 109
- WO-A-00/27297
- WO-A-95/13756
- FR-A- 2 696 091
- US-A- 5 042 982
- US-A- 5 520 689
- US-A- 5 643 260
- US-A- 6 090 111
- US-A- 6 110 172

## Beschreibung

Die Erfindung betrifft eine Pedikelschraube und ein Stützsystem gemäß Anspruch 1 bzw. 6.

Zur Stabilisierung von Wirbelsäulen sind invasive Behandlungsmethoden bekannt, bei denen zwischen benachbarten Wirbelkörpem unter Verwendung von Implantaten Knochenbrücken zum Wachsen gebracht werden. Durch die Knochenbrücken ergeben sich versteifende Fusionen der benachbarten Wirbel. Neben solchen Versteifungsoperationen ist auch eine Behandlungsmethode zur Stabilisierung mittels eines Implantatsystems bekannt, bei der keine Fusion entsteht, sondern eine Beweglichkeit zwischen benachbarten Wirbeln erhalten bleibt. Dieses Implantatsystem umfasst Pedikelschrauben und intervertebrale Stützelemente, die bei der Operation vom Rücken her implantiert werden. Jede Pedikelschraube hat einen als Ring ausgebildeten Kopf. Es werden jeweils zwei Pedikelschrauben in einem Wirbelkörper durch die Pedikelkanäle des Wirbels eingeschraubt. An den Schraubenköpfen werden die Stützelemerite befestigt. Zwei zwischen benachbarten Wirbeln parallel angeordnete Stützelemente bilden eine dynamische Abstützung dieser Wirbel. Die Wirbel - mit Ausnehme des untersten Lendenwirbels - können auch an den gleichen Pedikelschrauben jeweils mit einem oben sowie einem unten benachbarten Wirbel durch zwei Paare von Stützelementen verbunden werden.

Jedes Stützelement besteht aus einem Stück eines kabelartigen Bandes sowie einem zylindrischen Stützkörper, der eine elastische Nachgiebigkeit aufweist. Für einzelne Stützkörper kann auch ein starres Material verwendet werden, um die Wirbelsäule an einzelnen Stellen zu versteifen. Das Band ist in einem axialen Lumen des Stützkörpers eingezogen. Unter Verwendung des Bands werden die intervertebralen Stützelemente an den Pedikelschrauben befestigt. Dabei müssen die Bänder durch die Ringköpfe durchgezogen werden. Das Einziehen des Bands ist ein schwierig auszuführender Operationsschritt. Ein derartiges Stabilisierungssystem ist aus EP 0 669 109 B 1 bekannt.

Aufgabe der Erfindung ist es, für eine dynamische Wirbelabstützung eine Pedikelschraube für intervertebrale Stützelemente zu schaffen, für die das Befestigen des Stützelements an der Pedikelschraube, das während des chirurgischen Eingriffs erfolgen muss, erleichtert durchführbar ist. Diese Aufgabe wird durch die im Anspruch 1 definierte Pedikelschraube und durch das im Anspruch 6 definierte Stützsystem gelöst.

Die Pedikelschraube für intervertebrale Stützelemente besteht aus einem Schaft und einem mindestens zwei Teile umfassenden Kopf. Der Kopf ist als Befestigungsmittel für mindestens ein Stützelement ausgebildet. Jedes Stützelement besteht aus einem Stück eines kabelartigen Bandes sowie einem zylindrischen Stützkörper mit einem axialen, das Band enthaltenden Lumen. Das Band ist außerhalb von Endflächen des Stützkörpers im Kopf befestigbar. Der Kopf ist mit einer Kontaktfläche ausgebildet, über die eine Druckspannung auf den Stützkörper in Bandrichtung ausübbar ist, und zwar unter Verwendung des Bands sowie im Zusammenspiel mit einer weiteren Pedikelschraube. Ein Teil des Kopfes, der mit dem Schaft an dessen einen Ende fest verbunden ist, enthält eine zum Schaft transversal ausgerichtete Basisnut, in die beim Befestigen des Stützelements der zu befestigende Teil des Bandes oder ein das Band enthaltendes Verbindungsstück mittels einer translatorischen Bewegung in Richtung des Schafts einlegbar und dort fixierbar ist.

Die abhängigen Ansprüche 2 bis 5 betreffen vorteilhafte Ausführungsformen der erfindungsgemäßen Pedikelschraube.

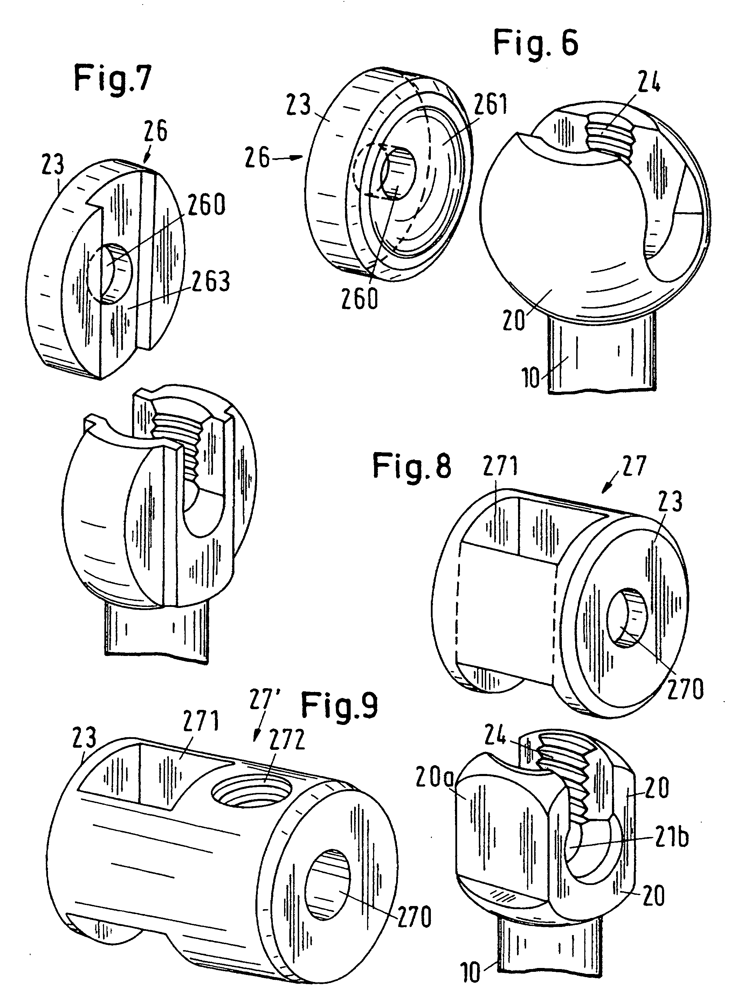

Nachfolgend wird die Erfindung anhand der Zeichnungen erläutert, in denen lediglich die Fig. 8 und 9 Lösungen gemäß der Erfindung zeigen, die übrigen Figuren jedoch für das Verständnis der Erfindung hilfreich sind.

Es zeigen:
- Fig. 1: einen Lendenwirbel mit Angabe der Position von zwei Pedikelschrauben,
- Fig. 2: zwei Pedikelschrauben mit einem intervertebralen Stützelement für zwei benachbarte Wirbel,
- Fig. 3: eine Umrissdarstellung von benachbarten Lendenwirbeln mit Angabe der Position von zwei implantierten Stützelementen,
- Fig. 4: den Kopf einer Pedikelschraube zusammen mit einem Stützelement,
- Fig. 5: einen Kopf mit einem T-Nutstein,
- Fig. 6, 7: zwei Köpfe, bei denen scheibenförmige Teile zwischen Stützelement und Kopf angeordnet werden,
- Fig. 8: einen Kopf mit Konnektor gemäß der Erfindung,
- Fig. 9: eine erfindungsgemäße Variante zum Konnektor der Fig. 8,
- Fig. 10: einen Kopf mit Überwurfteil,
- Fig. 11: einen Kopf mit einem anderen Konnektor und
- Fig. 12: einen Querschnitt durch den Kopf gemäß Fig. 10.

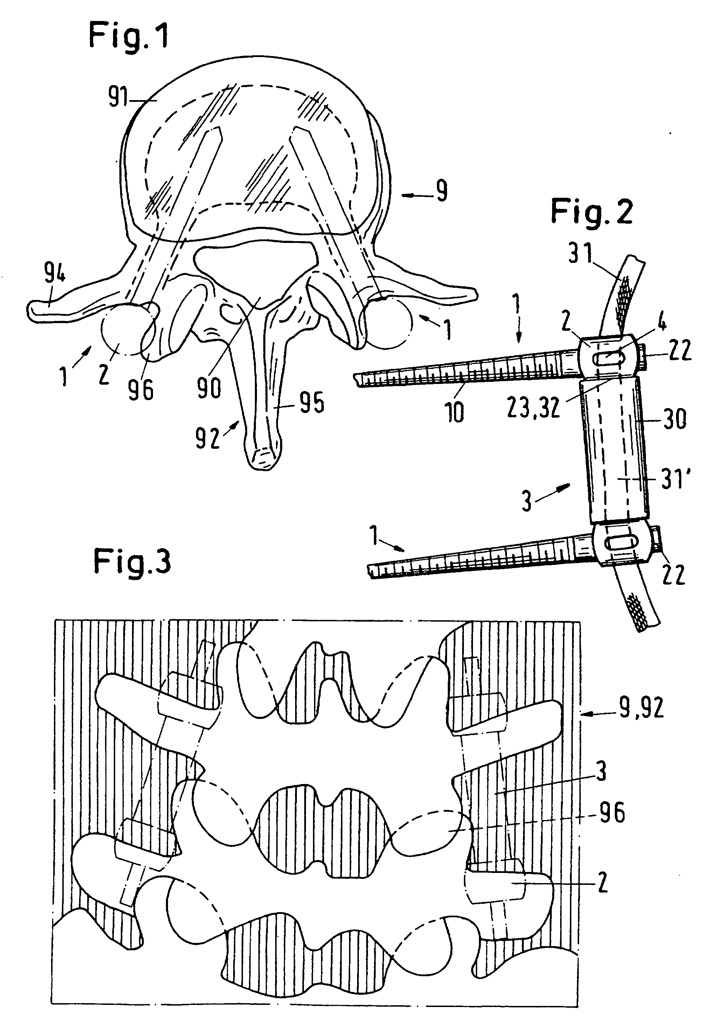

Ein Lendenwirbel 9 gemäß Fig. 1 besteht aus einem Wirbelloch 90, einem Wirbelkörper 91 und einem Wirbelbogen 92, der zwei Pedikel 93, zwei Querfortsätze 94, einen Dornfortsatz 95 und Gelenkfortsätze 96 umfasst. Strichpunktiert sind die Positionen von zwei Pedikelschrauben 1 im implantierten Zustand angedeutet. In diesen Positionen befinden sich Köpfe 2 der Pedikelschrauben 1 unmittelbar an der Außenseite der Pedikel 93 zwischen den Querfortsätzen 94 und den Gelenkfortsätzen 96. Fig. 2 zeigt zwei Pedikelschrauben 1, die für zwei benachbarte Wirbel 9 vorgesehen sind und zwischen deren Köpfe 2, die als Ringe ausgebildet sind, ein intervertebrales Stützelement 3 angeordnet ist. Schäfte 10 der Pedikelschrauben 1 werden in die Wirbel 9 eingeschraubt. Die Köpfe 2 weisen seitliche Kerben 4 auf, die für das Implantieren und Ausrichten der Pedikelschrauben 1 mittels eines Instruments benötigt werden. Das Stützelement 3 besteht aus einem Stück eines kabelartigen Bandes 31 und einem zylindrischen Stützkörper 30, in dem das Band 31 in einem axialen Lumen 31' enthalten ist. Das Band 31 ist außerhalb von Endflächen 32 des Stützkörpers 30 jeweils in den Köpfen 2 der Pedikelschrauben 1 mit einer Stiftschraube 22 befestigt. Die Köpfe 2 sind mit ebenen Kontaktflächen 23 ausgestattet, über die eine Druckspannung auf den Stützkörper 30 über deren Endflächen 32 in Bandrichtung ausübbar ist, wobei die dazu benötigte Zugkraft unter Verwendung des Bands 31 und im Zusammenspiel mit der Pedikelschraube 1 des benachbarten Wirbels 9 erzeugt wird.

In Fig. 3 ist eine Umrissdarstellung mit zwei benachbarten Lendenwirbeln 9 gezeigt. Es sind die Positionen zweier implantierter Stützelemente 3 angegeben.

Die Pedikelschraube 1 unterscheidet sich von anderen bekannten Pedikelschrauben durch einen besonders ausgebildeten Kopf 2. In diesen lässt sich das Stützelement 3 mittels einer translatorischen Bewegung in Richtung des Schafts 2 einlegen, so dass das Stützelement 3 im Kopf fixiert werden kann, ohne dass ein Einziehen des Bands 31 in ein ösenartiges Befestigungsmittel erforderlich ist.

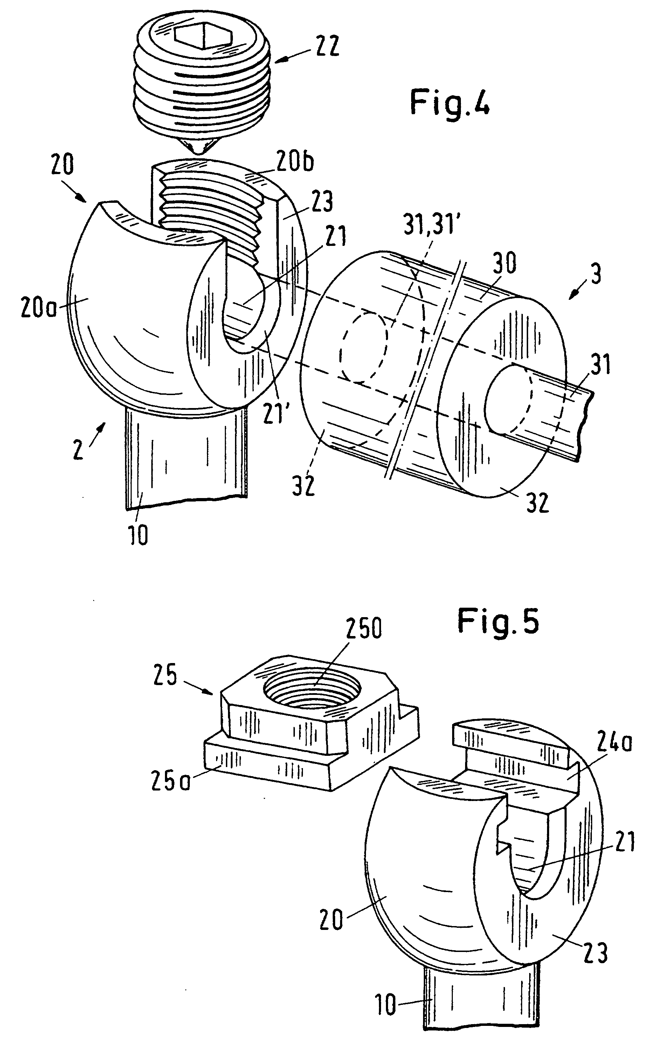

Ein Kopf 2 einer Pedikelschraube 1 ist in Fig. 4 zusammen mit dem Stützelement 3 dargestellt. Ein zwei Laschen 20a und 20b umfassender Teil 20 des Kopfes 2, der mit dem Schaft 10 fest verbunden ist, enthält eine zum Schaft 10 transversal ausgerichtete Basisnut 21 und ein Gewinde 24. In die Basisnut 21 wird beim Befestigen des Stützelements 3 ein zu befestigender Teil des Bandes 31 eingelegt und mit einer Schraube 22 fixiert. Die Basisnut 21 hat eine Form, die der Form des Bandes derart entspricht, dass die Basisnut im Einlegebereich komplementär ausgebildet ist. Eine Randzone 21' der Basisnut 21 kann - um einer Beschädigung des eingelegten Bandes 31 vorzubeugen - so ausgebildet sein, dass sich die Basisnut 21 gegen die Kontaktfläche 23 erweitert.

Fig. 4 zeigt ein erstes Beispiel für eine Pedikelschraube, bei der die Laschen 20a, 20b, welche Flanken der Basisnut 21 bilden, auf ihren Innenseiten Vertiefungen 24 aufweisen, in die ein Befestigungsteil, nämlich die Schraube 22 mittels eines Formschlusses verankerbar ist. Mit Vorteil werden die Laschen 20a, 20b nach Herstellen des Formschlusses durch das Befestigungsteil 22 elastisch etwas auseinander gespreizt, so dass dessen Lage zusätzlich aufgrund einer erhöhten Haftreibung und somit eines Kraftschlusses fixiert ist.

Ein zweites Beispiel ist in Fig. 5 gezeigt, bei dem die Laschen 20a, 20b auf ihren Innenseiten nutartige Vertiefungen 24a aufweisen. Ein T-Nutstein 25 mit seitlichen Rippen 25a wird nach Einlegen des Bandes 31 - vgl. Fig. 5 - zwischen die Nuten 24a eingeschoben und zum Fixieren des Bandes 31 eine Stiftschraube 22 - vgl. Fig. 5 - in dem Nutstein 25 in eine Bohrung mit Innengewinde 250 eingeschraubt.

Die Fig. 6 und 7 zeigen jeweils eine Kopf 2, bei dem ein scheibenförmiges Teil 26 zwischen Stützelement 3 und Kopf 2 angeordnet wird. Der Kopf 2 in Fig. 6 umfasst einen Kernteil 20 und mindestens ein scheibenförmiges Teil 26, das auf der einen Seite die Kontaktfläche 23 zum zylindrischen Stützkörper 30 des Stützelements 3 bildet und das auf der anderen Seite eine Kontaktfläche 261 zum Kernteil 20 bildet. Die Oberfläche des Kemteils 20 ist kugelförmig ausgebildet. Die Kontaktfläche 261 zum Kernteil 20 des scheibenförmigen Teils 26 ist der Form des Kernteils 20 entsprechend kalottenförmig ausgebildet. Das scheibenförmige Teil 26 wird zusammen mit dem Stützkörper 30 auf das Band 31 aufgebracht, wobei das Band 31 in die Bohrung 260 eingezogen wird. Dieses Einziehen des Bands 31 wird vor dem Implantieren des Stützelements 3 außerhalb des Körpers des zu operierenden Patienten durchgeführt.

Der Kopf 2 in Fig. 7 umfasst einen Kernteil 20, der an den Enden der Nut 21 je eine Erhebung 262 aufweist, die komplementär passend zu einer Nut 263 des scheibenförmigen Teils 26 ausgebildet ist (Kamm-Nut-Formschluss). Die Nut 263 lässt sich in Richtung des Schafts 10 auf die Erhebung 262 schieben. Wie bereits beim Beispiel der Fig. 6 wird das Teil 26 vor dem Implantieren auf das Band 31 aufgebracht.

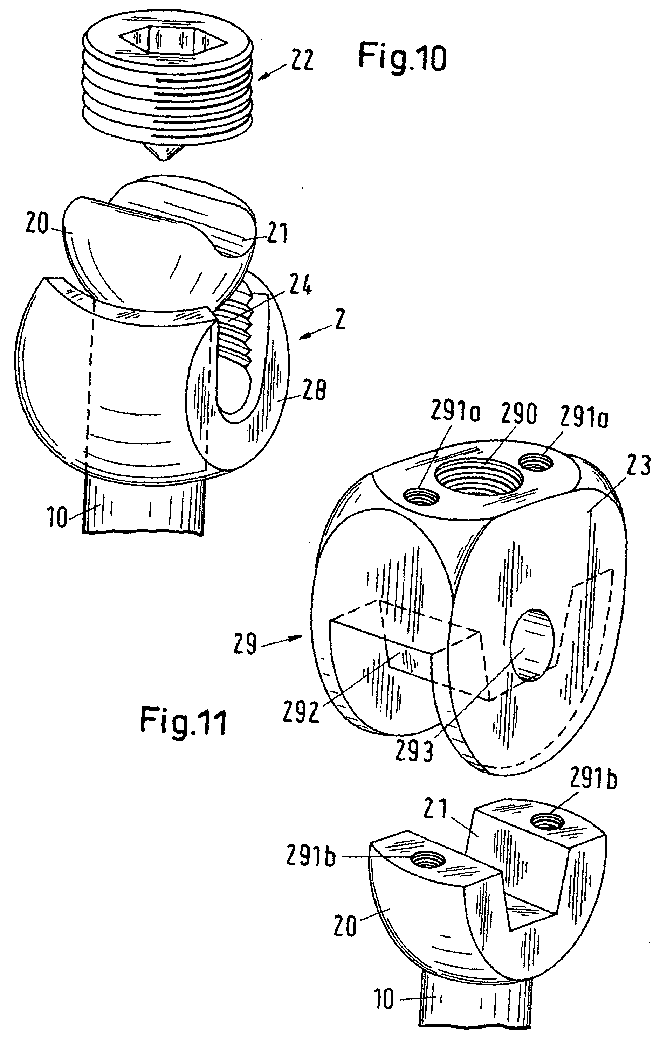

Fig. 8 zeigt einen Kopf 2 einer erfindungsgemäßen Pedikelschraube mit einem Kernteil 20 und mit einem Konnektor 27, der ein hülsenartiges Teil ist, dessen Innenraum ein von Planflächen gebildetes Vierkantprofil aufweist. Mit dem Konnektor 27 wird der Kernteil 20 dicht umschlossen, so dass ein Aufspreizen der Laschen 20a, 20b beim Einschrauben einer Schraube 22 (siehe Fig. 10) in das Gewinde 24 verhindert wird.

Fig. 9 zeigt eine erfindungsgemäße Variante 27' zum Konnektor 27 der Fig. 8. Dieser Konnektor 27' ist in Richtung der Nut 21 oder der Bohrung 270 länger ausgebildet und enthält eine Bohrung mit Innengewinde 272. Mit einer zweiten Schraube 22 kann das Band 31 zusätzlich unter der Gewindebohrung 272 fixiert werden.

Fig. 10 zeigt einen Kopf 2 mit einem Überwurfteil 28 und mit einem Kernteil 20, der mit dem einen Ende des Schafts 10 fest verbunden ist. Das Überwurfteil 28 ist längs des ganzen Schafts 10 bis zum genannten Schaftende verschiebbar. Dabei kommt das Gewinde 24 über die Nut 21 zu liegen, so dass die Schraube zum Befestigen des Bands 31 in das Gewinde 24 eingeschraubt werden kann.

Fig. 11 zeigt einen Kopf 2 mit einem Konnektor 29. Das Band 31 ist im Konnektor 29 in einer Bohrung 293 befestigbar. Während der Operation wird der Konnektor 29, der das Band 31 enthält, auf den Kernteil 20 des Kopfes 2 aufgesetzt, wobei ein Verbindungsstück 292 des Konnektors 29, das eine prismatische Form hat, in die entsprechend komplementär geformte Nut 21 eingelegt wird. Beispielsweise kann mit zwei Schrauben die Befestigung zwischen Konnektor 29 und Kernteil 20 hergestellt werden. Die nicht dargestellten Schrauben werden durch Bohrungen 291a des Konnektors 29 gesteckt und in Gewindebohrungen 291b des Kernteils 20 festgeschraubt.

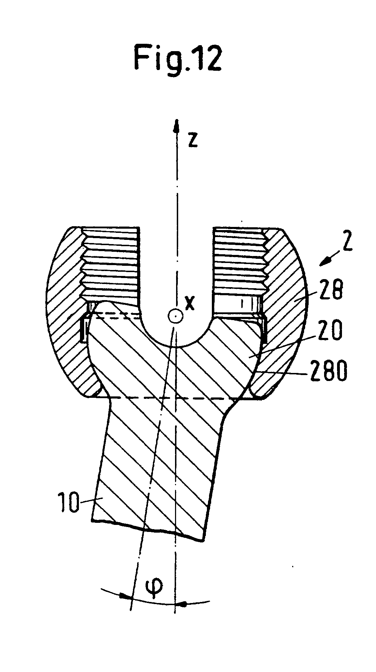

Die Ausführungen gemäß den Fig. 6 und 10 erlauben eine so genannte "Polyaxialität" beim Fixieren der Stützkörper 30 an den Pedikelschrauben 1. Die "Polyaxialität" betrifft die Achse des Schafts 2; diese steht bei allen anderen Ausführungsformen senkrecht zur Richtung der Stützkörper 30, die durch die Achse derer Lumen 31' gegeben ist. Mit "Polyaxialität" ist gemeint, dass der Winkel zwischen den beiden Achsrichtungen nicht fest vorgegeben ist, sondern in einem gewissen Winkelbereich variieren kann. Dies soll für die Ausführung gemäß Fig. 10 anhand der Fig. 12 näher erläutert werden: Fig. 12 zeigt eine Querschnittsdarstellung des Pedikelschraubenkopfs 20 mit dem Überwurfteil 28, dessen innere Oberfläche 280 ein Teilbereich einer Kugelfläche ist. Der Kopf 20 ist entsprechend kugelförmig ausgebildet, so dass er in verschiedenen Stellungen in den Überwurfteil 28 eingelegt werden kann. Die Achse des Schafts 10 hat eine Hauptrichtung z senkrecht zur Richtung x der Stützkörper 30 (x steht senkrecht zur Bildfläche). Die Schaftachse kann gegenüber dieser Hauptrichtung z um einen Winkel ϕ abweichen. Dieser Winkel ϕ hat einen maximalen Wert ϕₘₐₓ, der 10° oder auch mehr beträgt, aber kleiner als 15° ist. Der Winkel ϕ kann im Bereich eines Kegels um die Hauptrichtung z variieren, wobei der halbe Öffnungswinkel dieses Kegels ϕₘₐₓ beträgt. Entsprechendes gilt auch für die Ausführung gemäß der Fig. 6. Das System zur Wirbelabstützung, das mit den Pedikelschrauben gemäß den Fig. 6 und 10 hergestellt werden kann, ist dank der "Polyaxialität" leichter implantierbar.

Mit Pedikelschrauben werden Stützelemente umfassende Längskomponenten in Wirbeln verankert, wobei dank einer elastischen Nachgiebigkeit der Stützelemente eine dynamische Stabilisierung der Wirbel herstellbar ist. Die erfindungsgemäßen Pedikelschrauben erlauben eine Kopfzuführung der Längskomponenten. Bei der Kopfzuführung werden die Längskomponenten in die Köpfe der Pedikelschrauben eingelegt, indem lediglich eine translatorische Bewegung in Richtung des Schafts ausgeführt werden muss. Durch ein solches einfaches Einlegen vereinfacht sich offensichtlich die Operationstechnik wesentlich gegenüber der älteren Behandlungsmethode, bei der die Bänder der Stützelemente in die ringförmig ausgebildeten Schraubenköpfe eingezogen werden müssen.

## Patentansprüche

1. Pedikelschraube (1) mit einem Schaft (10) und einem mindestens zwei Teile (20, 22) umfassenden Kopf (2), wobei die Pedikelschraube (1) geeignet ist für intervertebrale Stützelemente (3), der Kopf (2) als Befestigungsmittel für mindestens ein Stützelement (3) ausgebildet ist, jedes Stützelement (3) aus einem Stück eines kabelartigen Bandes (31) sowie einem zylindrischen Stützkörper (30) mit einem axialen, das Band (31) enthaltenden Lumen (31') besteht, das Band (31) außerhalb von Endflächen (32) des Stützkörpers im Kopf (2) befestigbar ist und der Kopf (2) mit einer Kontaktfläche (23) ausgebildet ist, über die eine Druckspannung auf den Stützkörper (30) in Bandrichtung ausübbar ist, nämlich unter Verwendung des Bands (31) und im Zusammenspiel mit einer weiteren Pedikelschraube (1), wobei ein Teil (20) des Kopfes (2), der mit dem Schaft (10) an dessen einen Ende fest verbunden ist, eine zum Schaft (10) transversal ausgerichtete Basisnut (21) enthält, in die beim Befestigen des Stützelements (3) der zu befestigende Teil des Bandes (31) mittels einer translatorischen Bewegung in Richtung des Schafts (10) einlegbar und dort fixierbar ist,
**dadurch gekennzeichnet, dass** der die Basisnut (21) enthaltende, mit dem Schaft (10) verbundene Teil (20) des Kopfes (2) als Kernteil (20) ausgebildet ist und der Kopf (2) ein hülsenartiges, den Kernteil (20) dicht umschließendes Teil (27) umfasst, das mittels der translatorischen Bewegung in Richtung des Schaftes (10) auf den Kernteil (20) schiebbar ist.

2. Pedikelschraube nach Anspruch 1, **dadurch gekennzeichnet, dass** das Band (31) im Kopf (2) mit einer Schraube (22), insbesondere mit einer Zylinderschraube, fixierbar ist.

3. Pedikelschraube nach Anspruch 1 oder 2, **dadurch gekennzeichnet, dass** die Basisnut (21) eine Form hat, die der Form des Bandes (31) derart entspricht, dass die Basisnut im Einlegebereich komplementär ausgebildet ist.

4. Pedikelschraube nach einem der Ansprüche 1 bis 3, **dadurch gekennzeichnet, dass** die Basisnut (21) zwischen zwei Laschen (20a, 20b) in deren Basisbereich angeordnet ist und dass die Laschen auf ihrer Innenseite gewinde- oder nutförmige Vertiefungen (24; 24a) aufweisen, in die ein Befestigungsteil (22; 25), insbesondere eine Schraube (22), mittels eines Formschlusses verankerbar ist, wobei mit Vorteil die Laschen nach Herstellen des Formschlusses durch das Befestigungsteil elastisch etwas auseinander gespreizt sind, so dass dessen Lage zusätzlich aufgrund eines Kraftschlusses fixiert ist.

5. Pedikelschraube nach einem der Ansprüche 1 bis 4, **dadurch gekennzeichnet, dass** das hülsenartige Teil (27) als Konnektor ausgebildet ist und das Band (31) in dem Konnektor befestigbar ist, wobei der Konnektor auf dem Kernteil (20) des Kopfes (2) befestigbar ist.

6. Stützsystem mit wenigstens zwei Pedikelschrauben (1) nach einem der vorhergehenden Ansprüche und mit wenigstens einem intervertebralen Stützelement (3), wobei der Kopf (2) der Pedikelschrauben (1) jeweils als Befestigungsmittel für mindestens ein Stützelement (3) ausgebildet ist, jedes Stützelement (3) aus einem Stück eines kabelartigen Bandes (31) sowie einem zylindrischen Stützkörper (30) mit einem axialen, das Band (31) enthaltenden Lumen (31') besteht, das Band (31) außerhalb von Endflächen (32) des Stützkörpers im Kopf (2) befestigbar ist und der Kopf (2) mit einer Kontaktfläche (23) ausgebildet ist, über die eine Druckspannung auf den Stützkörper (30) in Bandrichtung ausübbar ist, nämlich unter Verwendung des Bands (31) und im Zusammenspiel mit einer weiteren Pedikelschraube (1).

## Claims

1. Pedicle screw (1), having a shaft (10) and a head (2) which includes at least two parts (20, 22), with the pedicle screw (1) being suitable for intervertebral support elements (3), the head (2) being formed as a securing means for at least one support element (3), each support element consisting of a piece of a cable-like band (31) and of a cylindrical support body (30) with an axial lumen (31') which contains the band (31), the band (31) being securable outside of end surfaces (32) of the support body in the head (2) and the head (2) being formed with a contact surface (23) via which a compressive stress can be exerted on the support body (30) in the band direction, namely using the band (31) and in collaboration with a further pedicle screw (1), wherein a part (20) of the head (2), which is firmly connected to the shaft (10) at the one end thereof, contains a base groove (21) which is oriented transversely to the shaft (10) and into which the part of the band (31) to be secured can be introduced on the securing of the support element (3) by means of a translatory movement in the direction of the shaft (10) and fixed there,
**characterized in that** the part (20) of the head (2) containing the base groove (21) and connected to the shaft (10) is made as a core part (20) and the head (2) includes a sleeve-like part (27) which tightly surrounds the core part (20) and which can be pushed in the direction of the shaft (10) onto the core part (20) by means of the translatory movement.

2. Pedicle screw in accordance with claim 1, **characterized in that** the band (31) can be fixed in the head (2) by a screw (22), in particular by a cylindrical screw.

3. Pedicle screw in accordance with claim 1 or claim 2, **characterized in that** the base groove (21) has a shape which corresponds to the shape of the band (31) such that the base groove is of complementary shape in the insertion region.

4. Pedicle screw in accordance with any one of the claims 1 to 3, **characterized in that** the base groove (21) is arranged between two lugs (20a, 20b) in their base region; and **in that** the lugs have thread-like or groove-like depressions (24; 24a) on their inner side into which a securing part (22; 25), in particular a screw (22), can be anchored by means of a shape matched connection, with the lugs advantageously being elastically spread apart somewhat by the securing part after the establishment of the shape matched connection such that the position of the securing part is additionally fixed as a result of a force transmitting connection.

5. Pedicle screw in accordance with any one of the claims 1 to 4, **characterized in that** the sleeve-like part (27) is made as a connector and the band (31) can be secured in the connector, with the connector being able to be secured to the core part (20) of the head (2).

6. A support system having at least two pedicle screws (1) in accordance with any one of the preceding claims and having at least one intervertebral support element (3), with the head (2) of the pedicle screws (1) in each case being formed as a securing means for at least one support element (3), each support element consisting of a piece of a cable-like band (31) and of a cylindrical support body (30) with an axial lumen (31') which contains the band (31), the band (31) being securable outside of end surfaces (32) of the support body in the head (2) and the head (2) being formed with a contact surface (23) via which a compressive stress can be exerted on the support body (30) in the band direction, namely using the band (31) and in collaboration with a further pedicle screw (1).

## Revendications

1. Vis pédiculaire (1) munie d'une tige (10) et d'une tête (2) comprenant au moins deux parties (20, 22), ladite vis pédiculaire (1) étant appropriée pour des éléments d'appui intervertébraux (3) ; la tête (2) étant réalisée sous la forme d'un moyen de fixation affecté à au moins un élément d'appui (3) ; chaque élément d'appui (3) étant composé d'un tronçon d'une bande (31) du type câble, ainsi que d'un corps cylindrique d'appui (30) comportant une cavité intérieure axiale (31') renfermant ladite bande (31) ; la bande (31) pouvant être fixée dans la tête (2), à l'extérieur de surfaces extrêmes (32) du corps d'appui ; et la tête (2) étant pourvue d'une surface de contact (23) par l'intermédiaire de laquelle une contrainte de pression peut être exercée sur le corps d'appui (30) dans la direction de la bande, plus précisément en utilisant ladite bande (31) et en coopération avec une autre vis pédiculaire (1), une partie (20) de la tête (2), reliée rigidement à la tige (10) à l'une des extrémités de cette dernière, comprenant une saignée de base (21) qui est orientée transversalement par rapport à ladite tige (10) et dans laquelle, lors de la fixation de l'élément d'appui (3), la partie de la bande (31) devant être fixée peut être insérée par un mouvement translatoire en direction de ladite tige (10), puis consignée à demeure dans ladite saignée,
**caractérisée par le fait que** la partie (20) de la tête (2), pourvue de la saignée de base (21) et reliée à la tige (10), est réalisée sous la forme d'une partie (20) formant noyau, et la tête (2) comprend une partie (27) en forme de douille qui ceinture hermétiquement ladite partie (20) formant noyau et peut coulisser, sur ladite partie (20) formant noyau, grâce au mouvement translatoire dans la direction de la tige (10).

2. Vis pédiculaire selon la revendication 1, **caractérisée par le fait que** la bande (31) peut être consignée à demeure dans la tête (2) au moyen d'une vis (22), notamment d'une vis cylindrique.

3. Vis pédiculaire selon la revendication 1 ou 2, **caractérisée par le fait que** la saignée de base (21) revêt une forme correspondant à la forme de la bande (31), de sorte que ladite saignée de base présente une réalisation complémentaire dans la zone d'insertion.

4. Vis pédiculaire selon l'une des revendications 1 à 3, **caractérisée par le fait que** la saignée de base (21) est interposée entre deux pattes (20a, 20b), dans la région de base de ces dernières ; et **par le fait que** lesdites pattes possèdent, sur leur face intérieure, des creusures (24 ; 24a) en forme de filetages ou de saignées dans lesquelles une pièce de fixation (22 ; 25), notamment une vis (22), peut être ancrée par concordance de formes, lesdites pattes étant avantageusement soumises à un léger écartement élastique provoqué par ladite pièce de fixation, après l'établissement de l'assemblage par conformation, si bien que la position de ladite pièce est additionnellement bloquée sous l'effet d'un assemblage mécanique.

5. Vis pédiculaire selon l'une des revendications 1 à 4, **caractérisée par le fait que** la partie (27) en forme de douille est réalisée sous la forme d'un organe de solidarisation, et la bande (31) peut être fixée dans ledit organe de solidarisation, ledit organe de solidarisation pouvant être fixé sur la partie (20) formant noyau de la tête (2).

6. Système d'appui comportant au moins deux vis pédiculaires (1) selon l'une des revendications précédentes, et au moins un élément d'appui intervertébral (3), la tête (2) des vis pédiculaires (1) étant respectivement réalisée sous la forme d'un moyen de fixation affecté à au moins un élément d'appui (3) ; chaque élément d'appui (3) étant composé d'un tronçon d'une bande (31) du type câble, ainsi que d'un corps cylindrique d'appui (30) comportant une cavité intérieure axiale (31') renfermant ladite bande (31) ; la bande (31) pouvant être fixée dans la tête (2), à l'extérieur de surfaces extrêmes (32) du corps d'appui ; et ladite tête (2) étant pourvue d'une surface de contact (23) par l'intermédiaire de laquelle une contrainte de pression peut être exercée sur le corps d'appui (30) dans la direction de la bande, plus précisément en utilisant ladite bande (31) et en coopération avec une autre vis pédiculaire (1).
